# EUROPEAN PATENT APPLICATION

(11) **EP 4 691 393 A1**
(43) Date of publication of application: **11.02.2026**
(21) Application number: 24803040.5
(22) Date of filing: 09.05.2024
(51) Int. Cl.: A61B 17/68

(54) **GUIDING AND LOCKING DEVICE, AND FRACTURE FIXATION SYSTEM COMPRISING SAME**

(30) Priority: 10.05.2023 CN 202310522652; 10.05.2023 CN 202321113175 U
(71) Applicant: Suzhou Kefu'er Medical Technology Co., Ltd, Suzhou, Jiangsu 215000 (CN)
(72) Inventor: XU, Daqiang, Suzhou, Jiangsu 215000 (CN)
(74) Representative: Gille Hrabal Partnerschaftsgesellschaft mbB Patentanwälte
(86) International application number: PCT/CN2024/091837
(87) International publication number: WO 2024/230761

(57) **Abstract**

Disclosed is a guided locking device and a fracture fixation system incorporating the same. The guided locking device comprises a guiding locking tube and a locking member. The guiding locking tube includes a locking end and a connecting end, and is provided with at least one elongated slot on its wall, the slot being in communication with the internal lumen and extending through the locking end. The locking member is configured to cause inward constriction of the tube wall at the locking end. The fracture fixation system comprises a bone plate, a Kirschner wire (K-wire), and the aforementioned guided locking device. The bone plate is provided with a fixation through-hole, to which the connecting end of the guiding locking tube is fixedly attached, such that the tube is arranged perpendicularly relative to the bone plate. The K-wire is received within the lumen of the guiding locking tube, and the locking member constricts the locking end inwardly to securely clamp the K-wire in position. This system reduces the risk of fracture displacement during K-wire fixation, avoids interference between the K-wire and the bone plate, and shortens the required intramedullary insertion length, thereby enabling unlimited application in intramedullary nail-assisted fracture fixation. The overall stability is superior to conventional multi-wire fixation techniques.

## Description

### Technical Field

The present invention relates to the technical field of medical fracture fixation. More particularly, it concerns a guided locking device and a fracture fixation system incorporating the same.

### Background

In fracture surgery, after reduction of the fracture, temporary fixation is required to allow fluoroscopic imaging from different angles in order to evaluate the quality of the reduction and to provide a foundation for definitive internal fixation using implants such as bone plates or intramedullary nails. The Kirschner wire (K-wire) is the most common means of temporary fracture fixation. In conventional procedures, multiple K-wires are inserted from one side of the fracture at appropriate sites and angles, advanced through the medullary cavity, and then penetrated across the fracture line to the opposite side. Although widely used, this approach has several limitations: 1) Considerable time and surgical expertise are required to secure the fracture fragments with K-wires. During insertion, additional forces are exerted on the fracture site, which remains unstable, making it difficult to maintain anatomical reduction. As a result, fracture displacement often occurs during the temporary fixation process. 2) The insertion of K-wires carries a risk of injury to surrounding vessels and nerves. 3) In some cases, the optimal K-wire trajectory for fixation interferes with the placement of a bone plate. Adjusting the trajectory to avoid interference compromises fixation stability. 4) As K-wires pass through the medullary cavity, they may obstruct subsequent placement of an intramedullary nail. Thus, conventional techniques are limited in scenarios requiring intramedullary fixation. 5) The stability achieved by K-wire fixation alone is relatively unreliable. During fluoroscopic evaluation of fracture reduction, or when repositioning or replacing implants such as intramedullary nails, bone plates, or external fixators, external forces may readily cause redisplacement of the fracture.

Accordingly, there is a demand for an improved device and system that provide secure temporary fixation while overcoming the limitations of existing techniques.

### Summary of the Invention

The purpose of the present invention is to address at least one of the aforementioned shortcomings in the prior art. To this end, a guided locking device and a fracture fixation system incorporating the same are provided.

In one aspect, the guided locking device comprises a guiding locking tube, formed as a hollow tubular structure with a locking end and a connecting end, and a locking member, disposed on the guiding locking tube. The wall of the guiding locking tube is provided with at least one elongated slot in communication with the internal lumen, the slot extending through the locking end. The locking member is configured to drive the wall of the locking end inwardly to achieve constriction.

In certain embodiments, the locking member is a hollow tubular component sleeved over the guiding locking tube and movable along its axial direction. During such movement, the locking member induces inward constriction of the locking end.

In certain embodiments, the lumen of the locking member comprises a cylindrical cavity and a conical cavity. The cylindrical cavity communicates with the smaller-diameter end of the conical cavity, and its diameter is less than the outer diameter of the locking end of the guiding locking tube. The locking member is sleeved over the locking end through the larger-diameter opening of the conical cavity and, when driven toward the connecting end, the combined action of the conical and cylindrical cavities produces inward constriction of the locking end.

In certain embodiments, the outer wall of the guiding locking tube is provided with a stepped structure. The elongated slot passes through this stepped structure. The locking member is positioned adjacent to the stepped structure, the outer diameter of which is greater than the inner diameter of the locking member. When the locking member is advanced toward the stepped structure, compressive interaction occurs, thereby constricting the wall of the locking end.

In certain embodiments, the locking member is threadably connected to the guiding locking tube.

In certain embodiments, a locking aperture is formed on the wall of the locking member in communication with its lumen. A locking element is housed in the aperture and can be actuated to press against the guiding locking tube, thereby securing the locking member in position relative to the guiding locking tube.

In certain embodiments, a retaining ring is sleeved on the guiding locking tube on a side of the locking member opposite the connecting end. When the locking member is driven away from the connecting end, it simultaneously drives the retaining ring, which then constricts the wall of the locking end inwardly.

In certain embodiments, a protruding engagement structure is formed on the outer wall of the locking end near its distal tip. The retaining ring is positioned between the locking member and the engagement structure. The wall of the locking end is deformable such that, when constricted, its outer diameter does not exceed the inner diameter of the retaining ring.

In certain embodiments, the locking member is a clamp-type structure sleeved on the guiding locking tube, and when tightened, it produces inward constriction of the locking end.

In certain embodiments, the wall at the connecting end of the guiding locking tube is provided with a threaded structure.

### Fracture Fixation System

In another aspect, the invention provides a fracture fixation system comprising a bone plate, a K-wire, and the guided locking device described above. The bone plate is provided with a fixation through-hole. The connecting end of the guiding locking tube is fixedly connected to the through-hole such that the tube is oriented perpendicular to the bone plate. The K-wire is inserted into the lumen of the guiding locking tube, and the locking member constricts the locking end inwardly to clamp the wire securely.

In certain embodiments, the K-wire is further provided with a limiting structure.

In certain embodiments, the system further includes a specialized tool comprising a handle and a tool shaft portion, one end of which is connected to the handle and the other end of which is provided with an actuating structure.

Compared with the prior art, the guided locking device and fracture fixation system of the present invention offer at least one of the following advantages:
1. Rapid and reliable preliminary stability: By placing the bone plate at the fracture site, inserting K-wires guided by the locking device on both sides, and locking them in place, a secure construct is quickly formed. This reduces operative time and technical difficulty while lowering the risk of fracture displacement during K-wire insertion.
2. Reduced surgical risk: The limiting structure on the K-wire allows precise control of insertion depth, reducing the likelihood of vascular and nerve injury.
3. Minimized secondary trauma: The wires are positioned away from the fracture line, avoiding additional injury to the most severely damaged region.
4. No interference with bone plate placement: The arrangement prevents conflict between the K-wire trajectory and the bone plate.
5. Compatibility with intramedullary fixation: Reduced intramedullary length of wire insertion enables unrestricted application in intramedullary nailing procedures. Multiple K-wires form a locked structure with the bone plate via the guided locking device, with only partial entry into the medullary cavity, providing effective temporary fixation. The limiting structure ensures controlled depth, avoiding obstruction of intramedullary nail placement.
6. Superior stability: The locked construct formed by the bone plate, multiple K-wires, and the guided locking device provides greater stability than conventional multi-wire fixation, reducing the risk of redisplacement during imaging or during placement or replacement of implants such as intramedullary nails, bone plates, or external fixators.
7. Facilitated implant positioning: The K-wires can serve as positioning guides for the bone plate and screws. Properly placed K-wires may be sequentially replaced with screws, while suboptimally placed wires provide reference for the correct alignment of the plate and screws.

### Brief Description of the Drawings

Figure 1 illustrates a schematic structural view of a bone plate according to an embodiment of the present disclosure.
Figure 2 illustrates a schematic structural view of a Kirschner wire according to an embodiment of the present disclosure.
Figure 3 illustrates a schematic structural view of a guided locking device according to an embodiment of the present disclosure.
Figure 4 illustrates a schematic structural view of a guiding locking tube according to an embodiment of the present disclosure.
Figure 5 illustrates a schematic structural view of a Kirschner wire having an external thread structure formed on a portion of the rod adjacent to the tip, according to an embodiment of the present disclosure.
Figure 6 illustrates a schematic structural view of a guiding locking tube having an elongated slot extending through both ends thereof, according to an embodiment of the present disclosure.
Figure 7 illustrates a schematic structural view of a locking member having a polygonal prism shape, according to an embodiment of the present disclosure.
Figure 8 illustrates a schematic structural view of a locking member having one end opposite the connecting end of the guiding locking tube formed as a polygonal prism, according to an embodiment of the present disclosure.
Figure 9 illustrates a half-sectional schematic view of the locking member shown in Figure 8.
Figure 10 illustrates a partial sectional schematic view of a guided locking device into which a Kirschner wire is inserted, according to an embodiment of the present disclosure.
Figure 11 illustrates a schematic structural view of a locking member provided with an internal thread structure, according to an embodiment of the present disclosure.
Figure 12 illustrates a schematic structural view of a locking member provided with a locking element, according to an embodiment of the present disclosure.
Figure 13 illustrates a schematic structural view of a guiding locking tube having a stepped structure formed on its outer wall, according to an embodiment of the present disclosure.
Figure 14 illustrates a partial sectional schematic view of a guided locking device when the guiding locking tube has the structure shown in Figure 13.
Figure 15 illustrates a half-sectional schematic view of a locking member whose lumen comprises a cylindrical cavity and a conical cavity, according to an embodiment of the present disclosure.
Figure 16 illustrates a schematic structural view of another guiding locking tube having a stepped structure formed on its outer wall, according to an embodiment of the present disclosure.
Figure 17 illustrates a schematic structural view of a guided locking device when the guiding locking tube has the structure shown in Figure 16.
Figure 18 illustrates a schematic structural view of a guided locking device with a Kirschner wire inserted, wherein the locking member is secured to the guiding locking tube by a locking element, according to an embodiment of the present disclosure.
Figure 19 illustrates a partial sectional schematic view of a guided locking device with a Kirschner wire inserted, wherein a retaining ring is provided, according to an embodiment of the present disclosure.
Figure 20 illustrates a partial sectional schematic view of a guided locking device with a Kirschner wire inserted, wherein a protruding engagement structure is formed on the outer wall of the locking end of the guiding locking tube near its distal portion, according to an embodiment of the present disclosure.
Figure 21 illustrates an enlarged schematic view of region I in Figure 20.
Figure 22 illustrates a schematic structural view of a guided locking device with a Kirschner wire inserted, wherein the locking member is a clamp, according to an embodiment of the present disclosure.
Figure 23 illustrates a schematic view of the mounting position of a limiting member on a Kirschner wire, according to an embodiment of the present disclosure.
Figure 24 illustrates a schematic view of the relative mounting position between a Kirschner wire with a limiting member and the guided locking device, according to an embodiment of the present disclosure.
Figure 25 illustrates a schematic structural view of a guiding locking tube provided with a polygonal prism-shaped second protruding structure, according to an embodiment of the present disclosure.
Figure 26 illustrates a schematic structural view of a dedicated tool provided with a polygonal prism-shaped limiting groove, according to an embodiment of the present disclosure.
Figure 27 illustrates a schematic structural view of a dedicated tool provided with a polygonal prism-shaped limiting protruding structure, according to an embodiment of the present disclosure.
Figure 28 illustrates a schematic structural view of a dedicated tool provided with a cross-shaped limiting protruding structure, according to an embodiment of the present disclosure.
Figure 29 illustrates a schematic view of the installation relationship between the guided locking device and the bone plate, according to an embodiment of the present disclosure.
Figure 30 illustrates a schematic view of the installation relationship between the guided locking device, the bone plate, and a target bone, according to an embodiment of the present disclosure.
Figure 31 illustrates a schematic view of the installation relationship between the fracture fixation system and a target bone, according to an embodiment of the present disclosure.

Reference numerals: 1 - guided locking device; 11 - guiding locking tube; 111 - locking end; 112 - connecting end; 113 - elongated slot; 114 - first threaded structure; 115 - stepped structure; 116 - protruding engagement structure; 117 - fifth threaded structure; 118 - sixth threaded structure; 119 - polygonal prism structure; 120 - second protruding structure; 12 - locking member; 121 - cylindrical cavity; 122 - conical cavity; 123 - first locking aperture; 124 - first locking element; 1241 - first groove structure; 125 - fourth threaded structure; 126 - seventh threaded structure; 127 - eighth threaded structure; 128 - receiving groove; 129 - stop surface; 13 - retaining ring; 131 - flange structure; 2 - bone plate; 21 - fixation through-hole; 211 - second threaded structure; 3 - Kirschner wire; 31 - tip portion; 32 - rod portion; 33 - third threaded structure; 4 - limiting member; 41 - second locking aperture; 42 - second locking element; 5 - dedicated tool; 51 - handle; 52 - tool shaft portion; 53 - actuating structure; 6 - bone.

### Detailed Description of the Embodiments

To further illustrate the technical solutions adopted in the present invention for achieving the intended objectives, detailed embodiments are described below with reference to the accompanying drawings. These embodiments describe the structure, features, and functions of the invention, but are not intended to limit its scope.

In one embodiment, a fracture fixation system is provided. The system comprises a bone plate 2, Kirschner wires (K-wires) 3, and at least one guided locking device 1.

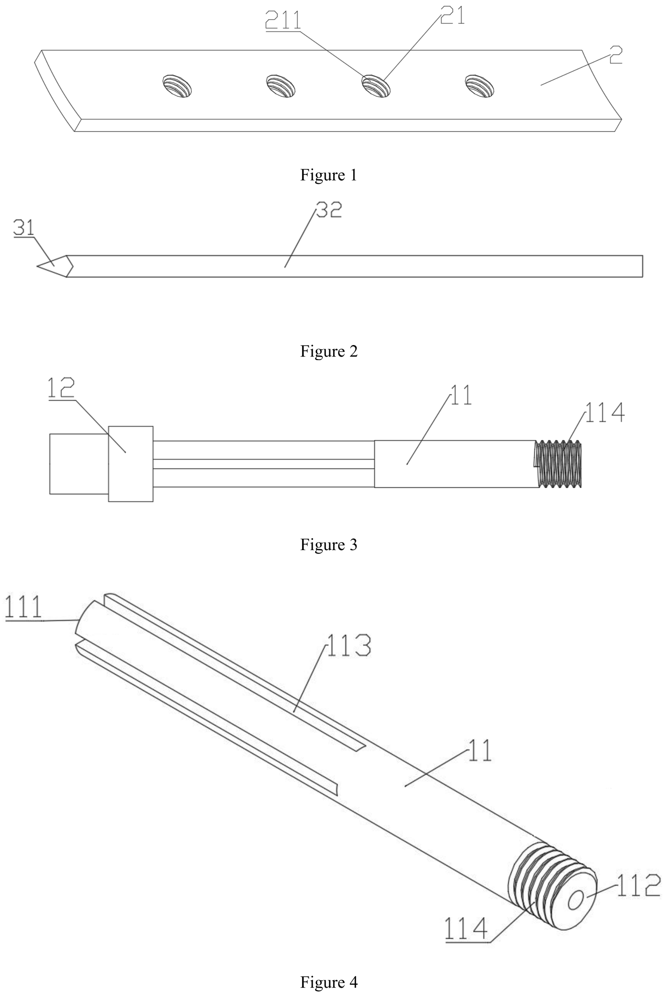

As shown in Figure 1, the bone plate 2 is provided with at least two fixation through-holes 21 arranged at intervals along its longitudinal axis. The fixation system includes at least two sets of guided locking devices 1 and K-wires 3, and preferably two sets are provided.

As shown in Figure 2, each K-wire 3 comprises a tip portion 31 and a rod portion 32 arranged along its axis. The tip portion 31 may be configured as a polyhedral pyramidal structure, such as triangular, quadrangular, or other polygonal forms. The distal apex of the tip portion 31 may be blunted to minimize the risk of injury to adjacent tissues of the bone 6 or sharpened to facilitate penetration into the bone 6. The K-wire 3 is advantageous due to its rapid insertion and minimal trauma to the bone 6. It facilitates re-reduction of the fracture and positional adjustment between the bone 6 and the bone plate 2, thereby improving surgical efficiency. For this reason, the K-wire 3 serves as an effective alternative to screws for temporary fixation.

As shown in Figure 3, the guided locking device 1 comprises a guiding locking tube 11 and a locking member 12. The guiding locking tube 11 is a hollow tubular structure having a locking end 111 and a connecting end 112 (Figure 4). The connecting end 112 of the locking end 111 is fixedly attached to a fixation through-hole 21 of the bone plate 2 such that the guiding locking tube 11 is oriented perpendicular to the surface of the bone plate 2. Preferably, the guiding locking tube 11 and the fixation through-hole 21 are connected by threaded engagement. Specifically, the wall of the guiding locking tube 11 near the connecting end 112 is provided with an external thread, defined as a first threaded structure 114. The wall of the fixation through-hole 21 is provided with a corresponding internal thread, defined as a second threaded structure 211. Engagement between the first threaded structure 114 and the second threaded structure 211 secures the connecting end 112 of the guiding locking tube 11 to the fixation through-hole 21. The K-wire 3 is disposed within the lumen of the guiding locking tube 11. The locking member 12 is configured to drive the wall of the locking end 111 inwardly to securely clamp the K-wire 3. The guided locking device 1 is preassembled onto the bone plate 2. The bone plate 2 is positioned over the fracture site of the target bone 6 and conforms to the bone surface. A first K-wire 3 is inserted into the guiding locking tube 11 of one guided locking device 1, with the tip portion 31 penetrating the bone 6 on one side of the fracture. A second K-wire 3 is inserted into the guiding locking tube 11 of the other guided locking device 1, with the tip portion 31 penetrating the bone 6 on the opposite side of the fracture. Once both K-wires 3 are clamped by their respective locking members 12, the bone plate 2 is secured to the bone 6. Through this arrangement, the guided locking device 1 and the bone plate 2 form a locked frame structure, which provides reliable temporary stabilization of the fracture and establishes a stable mechanical foundation for subsequent fixation procedures.

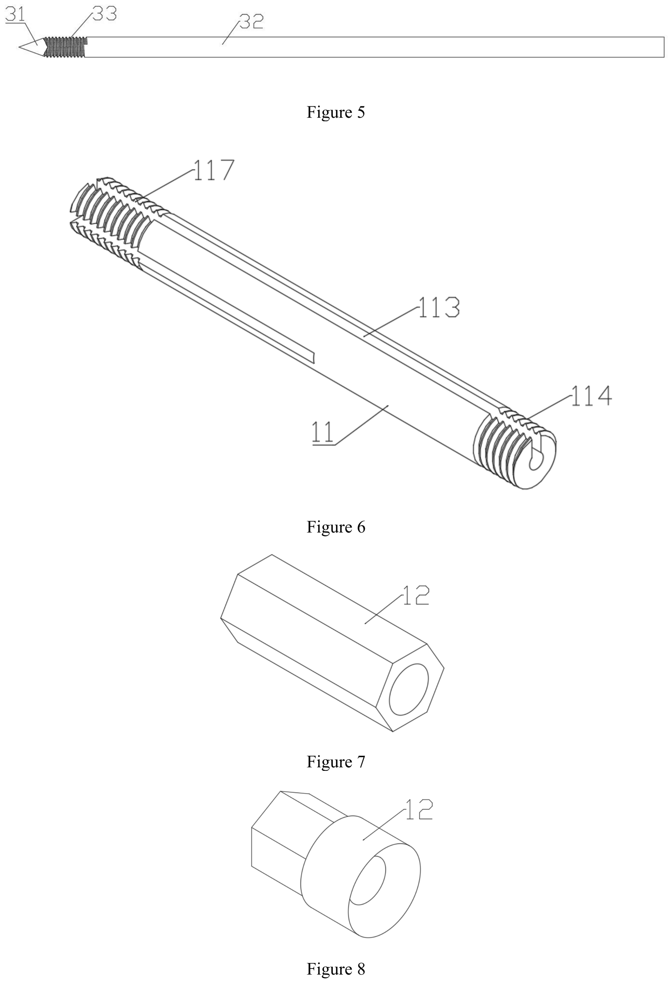

The surface of the K-wire 3 may be smooth or textured, with a textured surface being preferred to enhance clamping by the guiding locking tube 11. Further preferably, as shown in Figure 5, the rod portion 32 near the tip portion 31 may be provided with an external thread, defined as a third threaded structure 33. This configuration increases the anchoring effect of the K-wire 3 within the bone 6.

The wall of the guiding locking tube 11 is provided with at least one elongated slot 113, and preferably a plurality of such slots distributed circumferentially around the tube. Each elongated slot 113 communicates with the lumen of the guiding locking tube 11 and extends through the locking end 111 (Figure 4). Preferably, the slots 113 are aligned parallel to the longitudinal axis of the guiding locking tube 11. Alternatively, the slots may be oriented at an angle relative to the axis. The outer wall of the guiding locking tube 11 may be smooth or textured, preferably textured, to improve engagement with surrounding structures. Likewise, the inner wall may be smooth or textured, with a textured surface being preferred for enhanced clamping. In embodiments with a single elongated slot 113, the slot may extend only through the locking end 111, or through both the locking end 111 and the connecting end 112. In embodiments with multiple elongated slots 113, all slots may extend only through the locking end 111, or one slot may extend through both the locking end 111 and the connecting end 112, as shown in Figure 6.

In one embodiment, the locking member 12 is formed as a hollow tubular component sleeved over the guiding locking tube 11. The locking member 12 is configured to move axially along the guiding locking tube 11, and during such axial movement, the locking member 12 drives the wall of the locking end 111 of the guiding locking tube 11 to constrict inwardly. The outer surface of the locking member 12 may be smooth or textured, with a textured surface being preferred to enhance frictional engagement and tool manipulation. The radial cross-section of the locking member 12 is preferably at least partially non-circular, facilitating the use of a tool for rotational operation. For example, as shown in Figure 7, the locking member 12 may have an overall polygonal prism shape, or as illustrated in Figure 8, the end of the locking member 12 opposite the connecting end 112 of the guiding locking tube 11 may be formed as a polygonal prism. The incorporation of a non-circular section enables secure engagement with a wrench or specialized tool, thereby allowing precise rotation or axial advancement of the locking member 12. However, the shape of the locking member 12 is not limited to these examples and may adopt any suitable geometry.

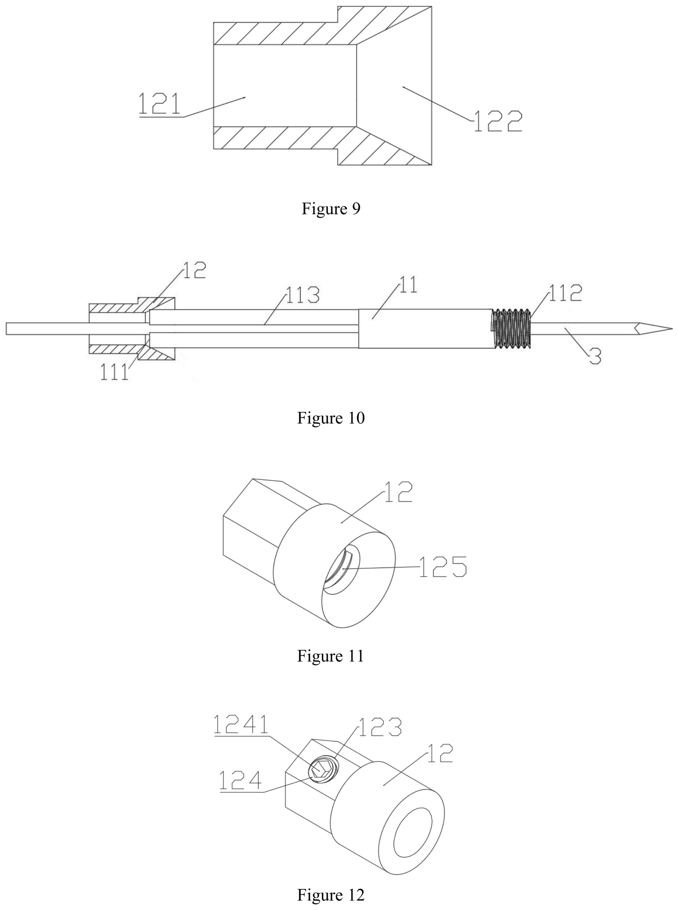

In another embodiment, the lumen of the locking member 12 comprises, along its axial direction, a cylindrical cavity portion 121 and a conical cavity portion 122, as shown in Figure 9. The cylindrical cavity portion 121 is in communication with the smaller-diameter end of the conical cavity portion 122. The internal diameter of the cylindrical cavity portion 121 is smaller than the outer diameter of the locking end 111 of the guiding locking tube 11. As illustrated in Figure 10, the locking member 12 is sleeved over the locking end 111 of the guiding locking tube 11 through the larger opening of the conical cavity portion 122. When the locking member 12 is driven toward the connecting end 112 of the guiding locking tube 11, the inner wall of the conical cavity portion 122 exerts a radial compressive force on the wall of the locking end 111, gradually constricting it inward. This inward constriction continues until the locking end 111 of the guiding locking tube 11 is received within the cylindrical cavity portion 121. The inward constriction of the locking end 111 produces a clamping action that securely holds the K-wire 3 within the lumen of the guiding locking tube 11, thus achieving stable mechanical fixation.

In a further embodiment, the locking member 12 may be connected to the guiding locking tube 11 via a direct press-fit engagement. As the locking end 111 is inserted into the cylindrical cavity portion 121, the inner wall of the cylindrical cavity portion 121 exerts radial pressure on the outer wall of the locking end 111. This pressure continues until the wall of the locking end 111 contacts and firmly clamps the K-wire 3 positioned within the guiding locking tube 11. Upon further advancement of the locking member 12, a tight frictional engagement is established between the cylindrical cavity portion 121 and the locking end 111, maintaining the locked condition. Preferably, the inner surface of the cylindrical cavity portion 121 is provided with a textured or roughened finish to increase the coefficient of friction, thereby improving the reliability and holding strength of the press-fit engagement between the locking member 12 and the guiding locking tube 11.

In another embodiment, the locking member 12 is threadably engaged with the guiding locking tube 11. As shown in Figure 11, the inner wall of the cylindrical cavity portion 121 is provided with an internal thread, defined as a fourth threaded structure 125. The outer wall of the guiding locking tube 11, adjacent to the locking end 111, is provided with a matching external thread, defined as a fifth threaded structure 117 (Figure 6). When the locking end 111 of the guiding locking tube 11 is received within the cylindrical cavity portion 121, rotation of the locking member 12 causes it to move axially relative to the guiding locking tube 11.

The engagement between the locking member 12 and the guiding locking tube 11 is not limited to press-fit or threaded connections. In yet another embodiment, a mechanical locking structure is employed. As illustrated in Figure 12, a locking aperture, defined as a first locking aperture 123, is formed in the wall of the locking member 12 and communicates with its internal lumen. A first locking element 124 is disposed within the first locking aperture 123 and is configured to be driven radially inward to press against the guiding locking tube 11, thereby securing the locking member 12 in place. Preferably, the first locking element 124 is implemented as a set screw. In this configuration, the first locking element 124 is threadably engaged with the wall of the locking aperture 123, allowing it to be advanced into the lumen of the locking member 12 to bear tightly against the outer surface of the guiding locking tube 11. This configuration allows the locking member 12 to be fixed in position with high precision while maintaining easy reversibility. The end of the set screw facing away from the lumen may include a tool engagement structure to facilitate rotation and tightening. A recessed feature, defined as a first groove structure 1241, which may be through or blind. The groove may have a polygonal, star, Torx, cruciform, or other suitable profile to receive a tool driver. Alternatively, a protruding feature may be provided, defined as a first protruding structure, which may similarly adopt a polygonal prism, star, Torx, or cruciform profile for tool engagement.

In a further embodiment, the outer wall of the guiding locking tube 11 is provided with a stepped structure 115. The elongated slot 113 extends through this stepped structure 115. The locking member 12 is disposed on one side of the stepped structure 115, and the outer diameter of the stepped structure 115 is greater than the inner diameter of the locking member 12. The locking member 12 is configured to be driven toward the stepped structure 115, and during such movement, the inner wall of the locking member 12 engages and compresses the stepped structure 115, thereby inducing inward constriction of the wall of the locking end 111 of the guiding locking tube 11.

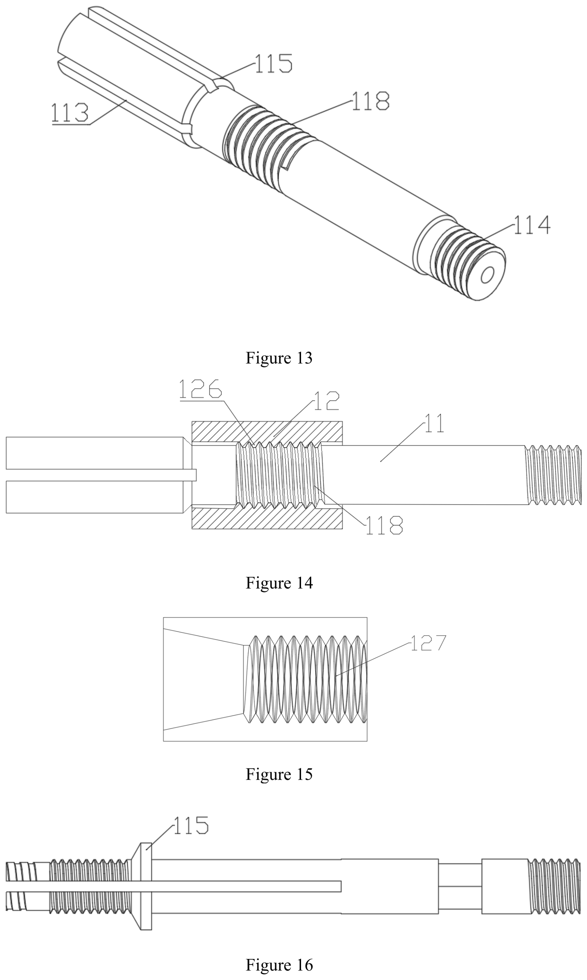

As shown in Figure 13, in one exemplary configuration, the locking end 111 of the guiding locking tube 11 is formed with an increased wall thickness, thereby creating a stepped structure 115 on the outer surface of the tube. The locking member 12 is sleeved over the thinner portion of the guiding locking tube 11. In this embodiment, the locking member 12 may have a simple cylindrical internal cavity (as shown in Figure 7). The side of the stepped structure 115 facing the connecting end 112 of the guiding locking tube 11 is preferably formed as an inclined transition surface. When the locking member 12 is driven toward the locking end 111 of the guiding locking tube 11, the inner wall of the locking member 12 contacts the inclined surface of the stepped structure 115, resulting in inward constriction of the wall of the locking end 111 of the guiding locking tube 11.. Preferably, the locking member 12 is threadably engaged with the guiding locking tube 11. As illustrated in Figures 13 and 14, the outer wall of the thinner portion of the guiding locking tube 11 is provided with an external thread, defined as a sixth threaded structure 118, while the inner wall of the locking member 12 is provided with a corresponding internal thread, defined as a seventh threaded structure 126. Engagement between the sixth threaded structure 118 and the seventh threaded structure 126 establishes a controlled threaded connection between the locking member 12 and the guiding locking tube 11. Rotation of the locking member 12 along the thread advances it axially along the guiding locking tube 11. In another embodiment, the locking member 12 comprises a composite internal cavity having a cylindrical cavity portion 121 and a conical cavity portion 122. When assembled, the locking member 12 is sleeved over the thinner section of the guiding locking tube 11 with the larger-diameter opening of the conical cavity portion 122 oriented toward the stepped structure 115. The side of the stepped structure 115 facing the connecting end 112 of the guiding locking tube 11 may be either planar or inclined, though an inclined transition surface is preferred. When the locking member 12 is driven toward the stepped structure 115, the inner wall of the conical cavity portion 122 exerts a constraining and compressive force on the stepped structure 115. This interaction causes inward constriction of the wall of the locking end 111. As shown in Figure 15, the locking member 12 is preferably threadably connected to the guiding locking tube 11. The inner wall of the cylindrical cavity portion 121 is provided with an internal thread, defined as an eighth threaded structure 127, which mates with the sixth threaded structure 118 formed on the outer wall of the guiding locking tube 11. Through engagement of the sixth threaded structure 118 and the eighth threaded structure 127, the locking member 12 can be advanced or retracted along the axial direction by rotational operation.

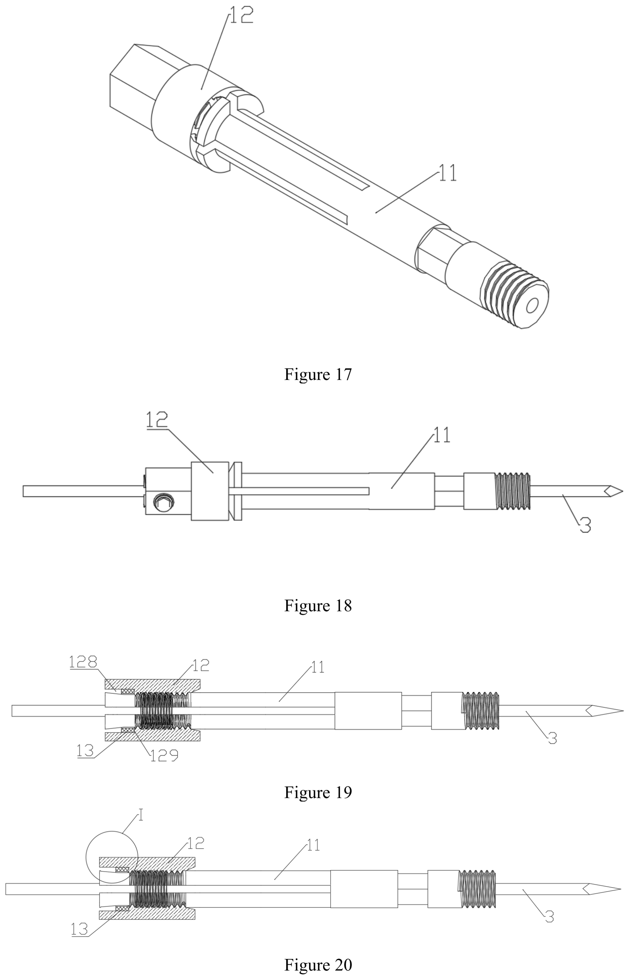

As shown in Figure 16, in another embodiment, the stepped structure 115 is formed as a circumferential protruding ring provided on the outer wall of the guiding locking tube 11 near the locking end 111. In this embodiment, the locking member 12 may be positioned on the side of the protruding structure facing away from the connecting end 112 (Figure 17) or on the side facing the connecting end 112 of the guiding locking tube 11. When the locking member 12 is driven toward the protruding structure, its inner wall engages and compresses the stepped structure 115, causing the wall of the locking end 111 of the guiding locking tube 11 to constrict inwardly and clamp the K-wire 3. Preferably, the locking member 12 is threadably engaged with the guiding locking tube 11. The lumen of the locking member 12 may be configured as a single cylindrical cavity, with the side of the protruding structure facing the locking member 12 formed as an inclined transition surface, or a composite cavity consisting of the cylindrical cavity portion 121 and the conical cavity portion 122, wherein the surface of the protruding structure facing the locking member 12 may be either inclined or planar. The operational principle of this embodiment corresponds to that described in the preceding examples and will not be repeated here.

It should be understood that in all embodiments incorporating the stepped structure 115, the connection between the locking member 12 and the guiding locking tube 11 is not limited to threaded engagement. Other connection mechanisms may also be utilized-for example, the mechanical locking arrangement employing a locking element as described in earlier embodiments (Figure 18). The structural principle and operational method of this configuration correspond to those previously described and will not be elaborated further for brevity.

In a further embodiment, the guiding locking tube 11 is provided with a retaining ring 13 positioned on the side of the locking member 12 opposite the connecting end 112 of the guiding locking tube 11. It should be noted that the retaining ring 13 is only applicable to the embodiments in which inward constriction of the wall of the locking end 111 of the guiding locking tube 11 is achieved by driving the locking member 12 toward the connecting end 112 of the guiding locking tube 11, as illustrated in Figure 19. The number of retaining rings 13 may vary according to design requirements, and one or more may be provided. When the locking member 12 is driven in the direction away from the connecting end 112 of the guiding locking tube 11, it pushes the retaining ring 13 in the same direction. As the retaining ring 13 moves along the outer wall of the guiding locking tube 11, its inner wall applies a circumferential compressive force on the wall of the locking end 111, thereby causing the tube wall to constrict radially inward. The internal diameter of the retaining ring 13 is designed to be slightly larger than the outer diameter of the locking end 111 of the guiding locking tube 11 when the elongated slots 113 are fully closed. As shown in Figures 19-21, the outer diameter of the wall of the locking end 111 of the guiding locking tube 11 gradually decreases toward the connecting end 112 of the guiding locking tube 11. During assembly, the locking member 12 is first driven toward the connecting end 112 of the guiding locking tube 11, compressing the wall of the locking end 111 of the guiding locking tube 11 inward until its distal outer diameter becomes smaller than the internal diameter of the retaining ring 13. The retaining ring 13 can then be sleeved over the locking end 111 of the guiding locking tube 11. Once the retaining ring 13 is in position, the locking member 12 is driven in the opposite direction-away from the connecting end 112 of the guiding locking tube 11. This movement causes the wall of the locking end 111 of the guiding locking tube 11 to elastically expand outward, increasing its outer diameter beyond the internal diameter of the retaining ring 13, thereby securing the retaining ring 13 on the locking end 111 of the guiding locking tube 11. As the locking member 12 continues to move away from the connecting end 112 of the guiding locking tube 11, it comes into contact with the retaining ring 13. Further advancement of the locking member 12 in this direction pushes the retaining ring 13 outward. Since the outer diameter of the locking end 111 of the guiding locking tube 11 increases progressively toward its distal extremity, the inner wall of the retaining ring 13 applies increasing radial compression on the locking end 111 of the guiding locking tube 11 during this movement, inducing additional inward constriction of the tube wall. This configuration enables bidirectional locking: driving the locking member 12 in either axial direction produces inward constriction of the locking end 111, allowing the device to clamp the K-wire 3 effectively from either movement direction. Such a design broadens the usability of the guided locking device 1 and enhances adaptability to different surgical manipulations and user preferences.

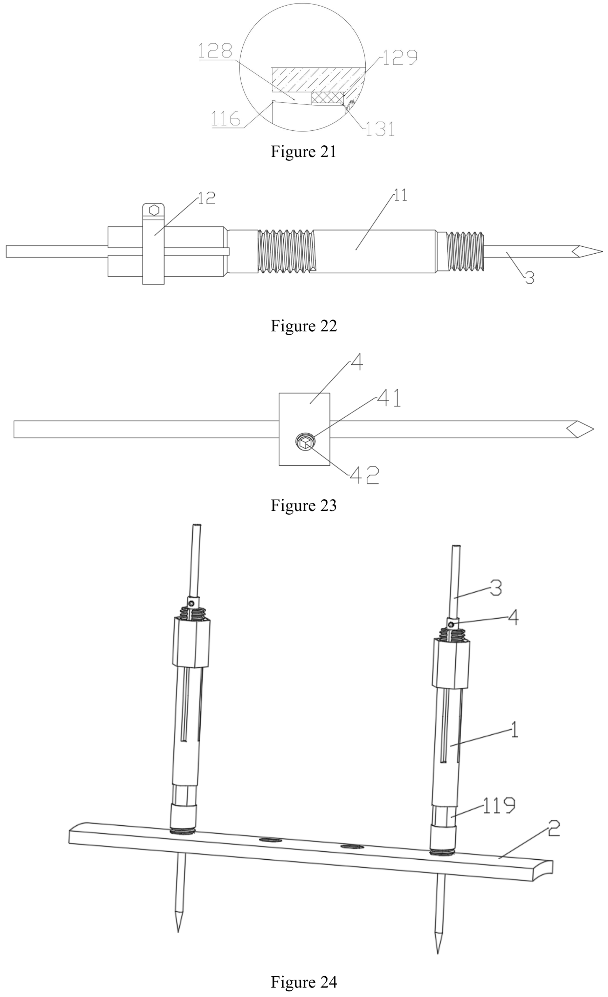

In another embodiment, as illustrated in Figures 20 and 21, a protruding engagement structure 116 is formed on the outer wall of the guiding locking tube 11 near the locking end 111. The retaining ring 13 is positioned between the locking member 12 and the protruding engagement structure 116. The wall of the locking end 111 of the guiding locking tube 11 can be constricted such that the outer diameter of the protruding engagement structure 116 becomes equal to or smaller than the internal diameter of the retaining ring 13. This ensures that the retaining ring 13 can be smoothly passed over the protruding engagement structure 116 and fitted onto the guiding locking tube 11 during assembly. As shown in Figure 21, a preferred retaining ring 13 comrpises an internal flange structure 131 defining its effective inner diameter. When the retaining ring 13 is located between the locking member 12 and the protruding engagement structure 116, the protruding engagement structure 116 acts as a stop surface for the flange structure 131. This configuration prevents the retaining ring 13 from detaching from the locking end 111 of the guiding locking tube 11 when the locking member 12 is driven away from the connecting end 112 of the guiding locking tube 11.

In another embodiment, as shown in Figures 19-21, the end of the locking member 12 opposite the connecting end 112 of the guiding locking tube 11 is provided with a retaining groove 128 configured to accommodate the retaining ring 13. A stop surface 129 is formed within the retaining groove 128 and is arranged to abut the retaining ring 13. When the locking member 12 is driven away from the connecting end 112 of the guiding locking tube 11, the stop surface 129 pushes the retaining ring 13 to move synchronously in the same direction. The provision of the retaining groove 128 allows the retaining ring 13 to be partially embedded within the locking member 12. This design not only ensures that the retaining ring 13 moves in concert with the locking member 12 during operation but also provides mechanical protection for the retaining ring 13.

In another embodiment, the locking member 12 is configured as a circumferential clamp. The clamp is sleeved over the portion of the guiding locking tube 11 in which the elongated slot 113 is formed. By tightening the clamp, circumferential compression is applied to the guiding locking tube 11, causing the wall of the locking end 111 to constrict radially inward. As illustrated in Figure 22, the clamp may be implemented as a band-type hose clamp. It should be understood that the clamp-type member is not limited to the configuration shown; other structures capable of producing circumferential tightening, such as flexible straps, may also be employed.

In a preferred embodiment, the locking end 111 of the guiding locking tube 11 is fabricated from a shape-memory alloy. In operation, after the K-wire 3 is inserted through the lumen of the guiding locking tube 11 and anchored into the bone 6, the locking end 111 of the guiding locking tube 11 is subjected to a specific temperature environment-for instance, immersion in cold water or exposure to a low-temperature medium. Under these conditions, the locking end 111 constricts inward and clamps the K-wire 3 securely, thereby achieving fixation. Preferably, the locking end 111 is composed of a thermoelastic shape-memory alloy. In some embodiments, only the locking end 111 is formed from shape-memory alloy material, while in other embodiments, the entire guiding locking tube 11 may be composed of the same alloy. The specific material selection is not limited herein, provided that the structure enables reliable clamping of the K-wire 3.

In a further embodiment, the fracture fixation system includes a limiting member 4, which is mounted on the K-wire 3 to control the depth of insertion. As shown in Figure 23, the limiting member 4 has an external dimension greater than the internal diameter of the guiding locking tube 11. When the K-wire 3 is inserted into the lumen of the guiding locking tube 11, the limiting member 4 abuts the distal edge of the locking end 111, as illustrated in Figure 24, thereby defining the maximum insertion length of the wire. The position of the limiting member 4 along the K-wire 3 may be adjusted according to surgical requirements, enabling precise control of the penetration depth of the K-wire 3 into the bone 6. The limiting member 4 is preferably formed as a hollow tubular body, with a cross-sectional shape that may be circular, polygonal, or of another suitable configuration. The inner wall of the limiting member 4 may be smooth or textured, with a textured surface being preferred. The wall of the limiting member 4 is provided with a locking aperture, defined as a second locking aperture 41, which communicates with its lumen. A second locking element 42 is disposed within the second locking aperture 41 and can be actuated to press against the K-wire 3, thereby locking the limiting member 4 in position. As shown in Figure 23, the second locking element 42 preferably adopts a set-screw configuration threadably engaged with the second locking aperture 41. Rotation of the set screw advances it into the cavity of the limiting member 4, where it presses against the surface of the K-wire 3 to achieve secure locking. The structure and function of the second locking element 42 correspond to those of the first locking element 124 described previously.

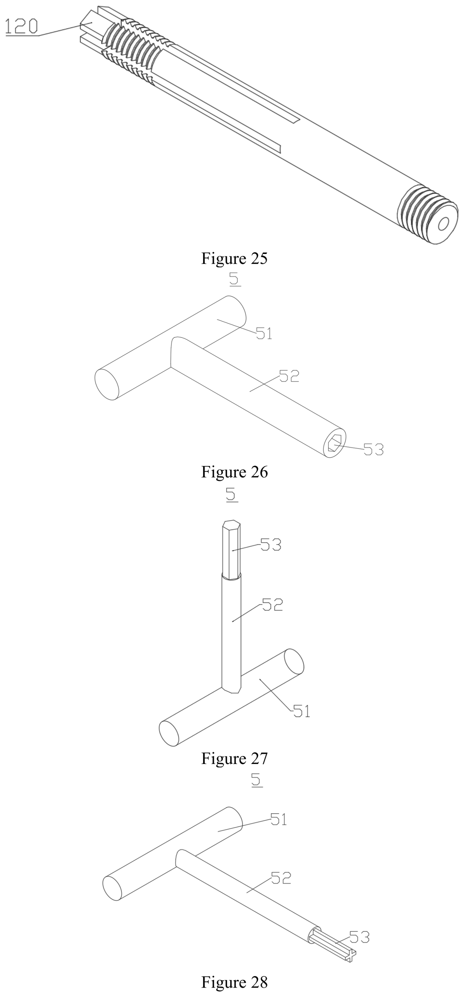

In another embodiment, portions of the guiding locking tube 11 are designed with non-circular cross sections to facilitate tool engagement and manipulation during assembly or adjustment. As shown in Figure 24, the section of the guiding locking tube 11 adjacent to the bone plate 2 may be formed with a polygonal prism structure 119. This feature allows engagement with a wrench or compatible tool for controlled rotation of the guiding locking tube 11
Additionally, the locking end 111 of the guiding locking tube 11 may include tool engagement structures configured to allow direct rotational actuation using specialized instruments. These may include a protruding structure, defined as a second protruding structure 120, which may have a polygonal, star-shaped, or cruciform geometry (Figure 25), or a recessed structure, defined as a second groove structure, which may similarly take polygonal, star, Torx, or cruciform shapes. Since the elongated slot 113 extends through the locking end 111, the opening of the slot itself may function as a second groove structure for tool engagement. Alternatively, a dedicated recess of a different geometry may be provided depending on operational requirements.

In a further embodiment, the fracture fixation system is equipped with a dedicated surgical tool 5, as illustrated in Figures 26-28. The tool 5 comprises a handle 51 and a tool rod 52, with one end of the tool rod 52 connected to the handle 51 and the opposite end provided with a working structure 53. The working structure 53 may be designed as a limiting groove structure matching the profile of the first protruding structure of the first locking element 124 (Figure 26) or as a limiting protruding structure configured to engage with the first groove structure 1241 of the first locking element 124 (Figures 27-28). During operation, the working structure 53 of the tool is engaged with the corresponding feature of the locking element 124, and rotation of the tool about its longitudinal axis drives the locking element 124 to tighten or release. The same method applies to the manipulation of the second locking element 42 on the limiting member 4.

In another embodiment, the working structure 53 may also be configured to engage with the distal end of the locking member 12 opposite the connecting end 112 of the guiding locking tube 11. The internal diameter of the limiting groove structure is greater than the size of the limiting member 4. When the limiting groove structure of the specialized tool 5 is fitted onto the locking member (12), axial rotation of the specialized tool 5 about its longitudinal axis correspondingly drives the rotation of the locking member 12.

In another embodiment, the working structure 53 may alternatively be formed as either a limiting groove structure whose shape corresponds to that of a second protruding structure 120 provided on the locking end 111 of the guiding locking tube (11) or a limiting protruding structure whose shape corresponds to that of a second groove structure provided on the locking end 111 of the guiding locking tube 11.. During use, the limiting groove structure of the specialized tool 5 may be engaged over the corresponding second protruding structure 120, or the limiting protruding structure of the specialized tool 5 may be inserted into the corresponding second groove structure. Upon axial rotation of the specialized tool 5, the guiding locking tube 11 is thereby rotated.

The fracture fixation system disclosed herein is adaptable for various types of fracture management and provides stable temporary fixation following anatomical reduction. An exemplary application of the system in a long-bone fracture procedure is described below with reference to Figures 23 and 29-31.

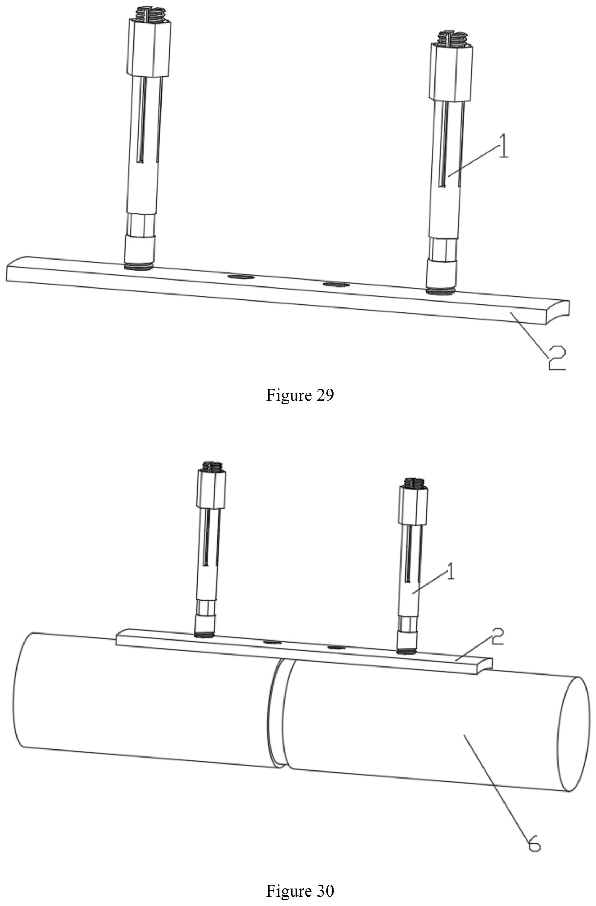

Step 1: Following standard sterile surgical protocols, the operative field is disinfected and draped. A bone plate 2 of appropriate size and contour is selected. Two guiding locking devices 1 are preassembled onto the bone plate 2, as illustrated in Figure 29. A limiting member 4 is then sleeved over each K-wire 3. The position of the limiting member 4 along the K-wire 3 is adjusted according to the desired penetration depth of the wire into the bone 6. Once positioned, the limiting member 4 is fixed to the K-wire 3, as shown in Figure 23.

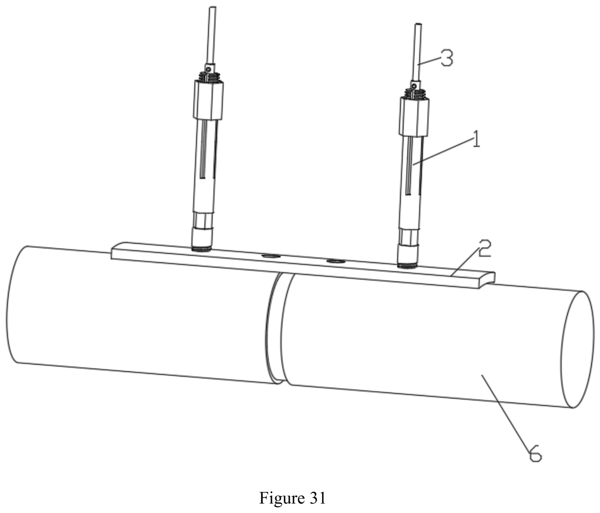

Step 2: Through a standard surgical approach, the fracture site is exposed. The bone plate 2 is positioned against the bone 6 at an appropriate location, as shown in Figure 30. A first K-wire 3 is inserted into the lumen of the guiding locking tube 11 of one guiding locking device 1. Using an electric drill, the wire is advanced into the bone 6 on one side of the fracture. The locking member 12 of the corresponding guiding locking device 1 is then rotated, causing the wall of the locking end 111 of the guiding locking tube 11 to contract radially inward and securely clamp the K-wire 3. Fracture alignment is achieved using traction or reduction forceps, and provisional reduction is maintained. A second K-wire 3 is then inserted through the guiding locking tube 11 of the opposite guiding locking device 1 and drilled into the bone 6 on the contralateral side of the fracture. The corresponding locking member 12 is rotated to achieve firm clamping of the second wire, as illustrated in Figure 31. The two K-wires 3, in conjunction with their respective guiding locking devices 1 and the bone plate 2, establish a locked structural framework that provides immediate provisional stability to the fracture site.

Step 3: Additional K-wires 3 may be introduced on both sides of the fracture line following the same procedure. Multiple fixation points create a robust temporary fixation construct, preventing displacement during further manipulation.

Step 4: The surgeon observes the quality of fracture reduction and verifies the positional relationship between the bone plate 2 and the bone 6 on both sides of the fracture through direct visualization and fluoroscopic imaging. If the reduction or plate position is suboptimal, the corresponding locking member 12 of the relevant guiding locking device 1 is loosened to release the clamped K-wire 3. The wire is then withdrawn using an electric drill. After re-reducing the fracture or repositioning the bone plate 2, a new K-wire 3 is inserted into the bone 6 through the guiding locking device 1 at an optimized position and reclamped by rotating the locking member 12. Fluoroscopic verification is repeated to verify the quality of the reduction and fixation. If adjustment remains necessary, the above steps are repeated until the fracture alignment and plate position are satisfactory.

Step 5: Once optimal reduction and plate positioning have been confirmed, each K-wire 3 and its corresponding guiding locking device 1 are sequentially removed. For each removal, a screw channel is drilled through the corresponding fixation through-hole 21 of the bone plate 2 into the bone 6 along the existing trajectory of the Kirschner wire 3. The drilling depth is measured, and a screw of appropriate diameter and length is inserted to replace the K-wire 3 and the guiding locking device 1. Additional screws may be implanted as required according to clinical judgment.

Step 6: Final fluoroscopic imaging is performed to confirm both satisfactory fracture alignment and proper positioning of the internal fixation construct. After thorough irrigation of the surgical field and hemostasis, the wound is closed in layers to complete the procedure.

Compared with the prior art, the guiding locking device and the fracture fixation system disclosed in the present application offer the following one or more significant advantages:
1. Rapid establishment of preliminary stability at the fracture site, reducing the risk of displacement during temporary fixation: Once the bone plate is positioned at the desired location on the target bone, K-wires are inserted into both sides of the fracture under the guidance of the guiding locking device. By tightening the locking member, the K-wires and the bone plate are rigidly coupled, thereby forming a stable structural construct within a short period. This mechanism simplifies intraoperative handling, reduces both operative time and surgical complexity, and minimizes the likelihood of fracture displacement during temporary fixation.
2. Reduction of intraoperative risks associated with K-wire placement: The inclusion of a limiting member on the K-wire allows precise control of the wire's penetration depth into the bone. This configuration reduces the risk of iatrogenic injury to surrounding neurovascular structures.
3. The system enables the K-wires to be placed away from the fracture ends, avoiding secondary damage to the area of greatest bone injury.

## Claims

1. A guided locking device, comprising a guiding locking tube (11) and a locking member (12), wherein the guiding locking tube (11) is a hollow tubular structure comprising a locking end (111) and a connecting end (112); at least one elongated slot (113) is formed on a wall of the guiding locking tube (11), the slot (113) being in communication with a lumen of the guiding locking tube (11) and extending through the locking end (111); the locking member (12) is disposed on the guiding locking tube (11) and is configured to drive the wall at the locking end (111) of the guiding locking tube (11) to constrict inwardly.

2. The device of claim 1, wherein the locking member (12) is a hollow tubular structure sleeved over the guiding locking tube (11); the locking member (12) is configured to move axially along the guiding locking tube (11), and during such movement, the locking member (12) drives the tube wall at the locking end (111) to constrict inwardly.

3. The device of claim 2, wherein the lumen of the locking member (12) comprises a cylindrical section (121) and a conical section (122) along an axial direction; the cylindrical section (121) communicates with a smaller-diameter end of the conical section (122); a diameter of the cylindrical section (121) is smaller than an outer diameter of the locking end (111) of the guiding locking tube (11); the locking member (12) is sleeved over the locking end (111) of the guiding locking tube (11) via a larger opening of the conical section (122), and is movable toward the connecting end (112) of the guiding locking tube (11) to progressively compress the locking end (111) of the guiding locking tube (11) inwardly by means of the conical (122) and cylindrical sections (121).

4. The device of claim 2, wherein a stepped structure (115) is formed on an outer wall of the guiding locking tube (11); the elongated slot (113) extends through the stepped structure (115); the locking member (12) is positioned adjacent to the stepped structure (115); an outer diameter of the stepped structure (115) is greater than an inner diameter of the locking member (12); as the locking member (12) is advanced toward the stepped structure (115), the stepped structure (115) is compressed by the locking member (12), thereby causing the tube wall at the locking end (111) to constrict inwardly.

5. The device of claim 3 or 4, wherein the locking member (12) is threadably connected to the guiding locking tube (11).

6. The device of claim 3 or 4, wherein a locking aperture is formed on the wall of the locking member (12) and communicates with the internal lumen thereof; a locking element is disposed within the locking aperture and is configured to be actuated to press against the outer wall of the guiding locking tube (11), thereby securing the positions of the locking member (12) and the guiding locking tube (11).

7. The device of claim 3 or 4, further comprising a retaining ring (13) sleeved onto the guiding locking tube (11), wherein the retaining ring (13) is disposed on a side of the locking member (12) opposite the connecting end (112); as the locking member (12) is moved away from the connecting end (112), it pushes the retaining ring (13) accordingly; during this movement, the retaining ring (13) drives the tube wall at the locking end (111) to constrict inwardly.

8. The device of claim 7, wherein a protruding engagement structure (116) is formed on the outer wall of the locking end (111) near its distal extremity; the retaining ring (13) is disposed between the locking member (12) and the protruding engagement structure (116); the wall of the locking end (111) is deformable such that, when constricted, its outer diameter is less than or equal to the inner diameter of the retaining ring (13).

9. The device of claim 1, wherein the locking member (12) is a clamp-type structure sleeved over the guiding locking tube (11), and is configured to constrict the locking end (111) inwardly when actuated.

10. The device of claim 1, wherein the wall of the connecting end (112) of the guiding locking tube (11) is provided with a threaded structure.

11. The device of claim 1, wherein the locking end (111) of the guiding locking tube (11) is made from a shape-memory alloy.

12. A fracture fixation system, comprising a bone plate (2), a K-wire (3), and a guided locking device (1) as defined in any one of claims 1 to 11, wherein the bone plate (2) is provided with at least one fixation through-hole (21); a connecting end (112) of the guiding locking tube (11) is fixedly connected to the fixation through-hole (21); the guiding locking tube (11) is oriented perpendicular to the bone plate (2); the K-wire (3) is inserted into the lumen of the guiding locking tube (11); the locking member (12) is configured to constrict the wall at the locking end (111) inwardly to securely clamp the K-wire (3).

13. The system of claim 12, wherein a limiting structure (4) is disposed on the K-wire (3).

14. The system of claim 12, further comprising a specialized tool (5), wherein the dedicated tool (5) comprises a handle (51) and a tool shaft (52); a first end of the tool shaft (52) is connected to the handle (51), and a second end is provided with an actuating structure (53).
